(19) Europäisches Patentamt
European Patent Office
Office européen des brevets

(11) Veröffentlichungsnummer: **0 144 086**
**A2**

(12) # EUROPÄISCHE PATENTANMELDUNG

(21) Anmeldenummer: 84114552.7

(22) Anmeldetag: 30.11.84

(51) Int. Cl.⁴: **C 07 J 5/00**
A 61 K 31/57

(30) Priorität: 08.12.83 DE 3344834

(43) Veröffentlichungstag der Anmeldung:
12.06.85 Patentblatt 85/24

(84) Benannte Vertragsstaaten:
AT BE CH DE FR GB IT LI LU NL SE

(71) Anmelder: SCHERING AKTIENGESELLSCHAFT Berlin und Bergkamen
Müllerstrasse 170/178 Postfach 65 03 11
D-1000 Berlin 65(DE)

(72) Erfinder: Annen, Klaus, Dr.
Osthofstrasse 80
44-Münster-Albachten(DE)

(72) Erfinder: Laurent, Henry, Dr.
Glambecker Weg 21
D-1000 Berlin 28(DE)

(72) Erfinder: Hofmeister, Helmut, Dr.
Weislingenstrasse 4
D-1000 Berlin 28(DE)

(72) Erfinder: Töpert, Michael, Dr.
Sigismundkorso 58
D-1000 Berlin 28(DE)

(54) **Neue Hydrocortison- und Prednisolon-Derivate.**

(57) Hydrocortison- bzw. Predinisolon-Derivate der allgemeinen Formel I

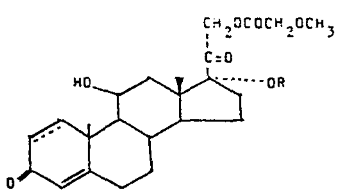

(I),

worin
..... eine Einfachbindung oder eine Dippelbindung R ein 1-Oxoalkylrest mit 2 bis 6 Kohlenstoffatomen, ein Benzoylrest oder ein Alkoxycarbonylrest darstellen, sind pharmakologisch wirksame Substanzen.

EP 0 144 086 A2

Die Erfindung betrifft den in den Patentansprüchen gekennzeichneten Gegenstand.

Die neuen Hydrocortison- und Prednisolon-Derivate der allgemeinen Formel I gemäß Patentanspruch 1 können als 2 bis 6 Kohlenstoffatome enthaltende 1-Oxoalkylgruppe R beispielsweise eine Acetylgruppe, eine Propionylgruppe, eine Butyrylgruppe, eine Isobutyrylgruppe, eine Valerylgruppe, eine 3-Methylbutyrylgruppe, eine Trimethylacetylgruppe, eine Hexanoylgruppe oder eine Äthoxycarbonylgruppe tragen.

Es wurde gefunden, daß die im Anspruch 1 gekennzeichneten Derivate des Hydrocortisons und Prednisolons überraschenderweise bei topischer Applikation eine signifikant starke Wirksamkeit besitzen; diese Wirksamkeit ist oft sogar noch signifikant stärker als diejenige fluorierter hochaktiver Kortikoide, wie etwa das 6α,9α-Difluor-11β-hydroxy-16α-methyl-21-valeryloxy-1,4-pregnadien-3,20-dion (=Nerisona).

Bei systemischer Applikation sind diese Derivate des Hydrocortisons und Prednisolons überraschenderweise schwächer wirksam als entsprechende vorbekannte Derivate des Hydrocortisons bzw. Prednisolons.

Die neuen Hydrocortison-Derivate der allgemeinen Formel I gemäß Patentanspruch 1 eignen sich demzufolge in Kombination mit den in der galenischen Pharmazie üblichen Trägermitteln zur lokalen Behandlung von Kontaktdermatitis, Ekzemen der verschiedensten Art, Neurodermatosen, Erythodermie, Verbrennungen, Pruritis vulvae et ani, Rosacea, Erythematodes cutaneus, Psoriasis, Lichen ruber planus et verrucosus und ähnlichen Hauterkrankungen.

Die Herstellung der Arzneimittelspezialitäten erfolgt in üblicher Weise, indem man die Wirkstoffe mit geeigneten Zusätzen in die gewünschte Applikationsform, wie zum Beispiel: Lösungen, Lotionen, Salben, Cremes oder Pflaster, überführt. In den so formulierten Arzneimitteln ist die Wirkstoffkonzentration von der Applikationsform abhängig. Bei Lotionen, Salben und Cremes wird vorzugsweise eine Wirkstoffkonzentration von 0,001 % bis 1 % verwendet.

Darüber hinaus sind die neuen Verbindungen gegebenenfalls in Kombination mit den üblichen Trägermitteln und Hilfsstoffen auch gut zur Herstellung von Inhalationsmitteln geeignet, welche zur Therapie allergischer Erkrankungen der Atemwege, wie zum Beispiel des Bronchialasthmas oder der Rhinitis, verwendet werden können.

Ferner eignen sich die neuen Kortikoide auch in Form von Kapseln, Tabletten oder Dragees, die vorzugsweise 10 bis 200 mg Wirkstoff enthalten und oral appliziert werden oder in Form von Suspensionen, die vorzugsweise 100 bis 500 mg Wirkstoff pro Dosiseinheit enthalten und rektal appliziert werden, auch zur Behandlung entzündlicher Erkrankungen des Darmtraktes, wie der Kolitis ulcerosa und der Kolitis granulomatosa.

Die neuen Hydrocortison- und Prednisolon-Derivate können nach dem in dem Patentanspruch 16 gekennzeichneten Verfahren hergestellt werden, welches unter den Bedingungen durchgeführt werden kann, die man üblicherweise zur Veresterung von 21-Hydroxysteroiden verwendet, siehe zum Beispiel die deutschen Patentanmeldungen Nr. 16 18 599, 26 45 104, 26 45 105 und 23 40 591 und 19 58 549 sowie das US-Patent Nr. 3,383,394. Die Synthesen der Ausgangsverbindungen werden in den Patentanmeldungen DE 26 45 105, EP 54786, EP 72547 und EP 742 beschrieben. Das bisher unbekannte 11ß,21-Dihydroxy-17α-propionyloxy-1,4-pregnadien-3,20-dion konnte analog DE 26 45 105 aus Prednisolon über den entsprechenden 17,21-Orthoester und dessen gepufferter Spaltung hergestellt werden.

Die nachfolgenden Ausführungsbeispiele dienen zur Erläuterung des erfindungsgemäßen Verfahrens.

Beispiel 1

Eine Lösung von 2,77 g (6.4 mMol) 17α-Butyryloxy-11ß.21-dihydroxy-4-pregnen-3.20-dion in 28 ml Pyridin wird bei 0°C mit 1.1 ml (11.9 mMol) Methoxyessigsäurechlorid tropfenweise versetzt und 1 Stunde unter Eisbadkühlung gerührt. Nach der Eiswasser-Kochsalzfällung filtriert man ab und nimmt den Rückstand in Methylenchlorid auf. Die organische Lösung wird neutralgewaschen, über Natriumsulfat getrocknet und im Vakuum eingeengt. Das Rohprodukt wird Aceton/Hexan kristallisiert. Man isoliert 1,76 g 17α-Bütyryloxy-11ß-hydroxy-21-methoxycetoxy-4-pregnen-3,20-dion. Schmelzpunkt 76-78 °C. $[\alpha]_D$ = +81° (Chloroform).

Beispiel 2

Unter den gleichen Bedingungen wird aus 11ß.21-Dihydroxy-17α-propionyloxy-1.4-pregnadien-3.20-dion [Fp. 221-223 °C; $[\alpha]_D$ = +53.3° (Pyridin)] das 11ß-Hydroxy-21-methoxyacetoxy-17α-propionyloxy-1.4-pregnadien-3.20-dion als Schaum mit $[\alpha]_D$ = +47° (Chloroform)erhalten.

Beispiel 3

Unter den gleichen Bedingungen wird aus 17α-Butyryloxy-11ß.21-dihydroxy-1.4-pregnadien-3.20-dion das 17α-Butyryloxy-11ß-hydroxy-21-methoxyacetoxy-1.4-pregnadien-3.20-dion mit dem Schmp. 87-88 °C und $[\alpha]_D$ = +47° erhalten.

0144086

## Patentansprüche

1. Hydrocortison-Derivate der allgemeinen Formel I

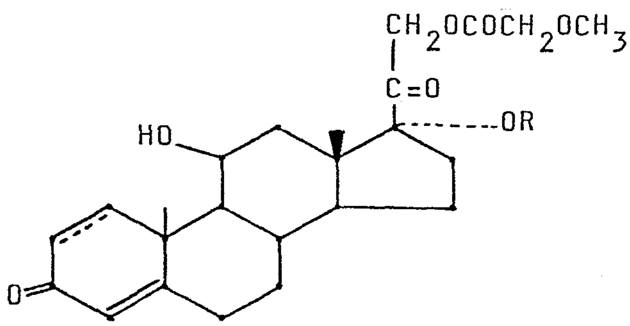

(I),

worin

..... eine Einfachbindung oder eine Doppelbindung und

R ein 1-Oxoalkylrest mit 2 bis 6 Kohlenstoffatomen ein Benzoylrest oder ein Alkoxycarbonylrest darstellen.

2. 17α-Butyryloxy-11β-hydroxy-21-methoxyacetoxy-4-pregnen-3,20-dion.

3. 11β-Hydroxy-21-methoxyacetoxy-17α-propionyloxy-1,4-pregnadien-3,20-dion.

4. 17α-Butyryloxy-11β-hydroxy-21-methoxyacetoxy-1,4-pregnadien-3,20-dion.

5. Pharmazeutische Präparate gekennzeichnet durch den Gehalt an ein oder zwei Hydrocortison-bzw. Prednisolon-Derivaten gemäß Anspruch 1 bis 4.

6. Verfahren zur Herstellung von Hydrocortison- bzw. Prednisolon-Derivaten der allgemeinen Formel I

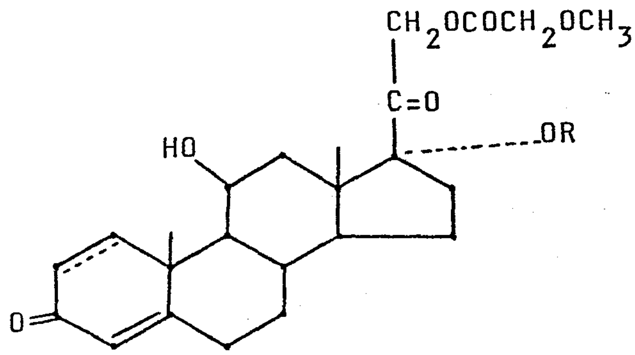

(I),

worin

..... und R die im Anspruch 1 genannte Bedeutung besitzen, dadurch gekennzeichnet, daß man in an sich bekannter Weise ein Hydrocortison-bzw. Prednisolon-17-acylat der allgemeinen Formel II

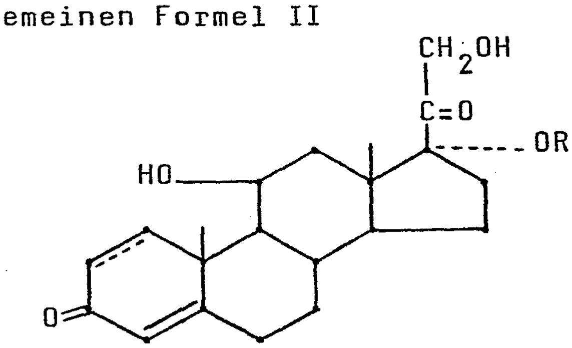

(II),

worin

..... und R die obengenannte Bedeutung besitzen, in der 21-Position mit Methoxyessigsäure oder einem reaktionsfähigen Derivat derselben verestert.